# NEUE EUROPÄISCHE PATENTSCHRIFT

(11) **EP 1 149 823 B2**
(45) Veröffentlichungstag und Bekanntmachung des Hinweises auf die Entscheidung über den Einspruch: **11.01.2006**
(45) Hinweis auf die Patenterteilung: 07.08.2002
(21) Anmeldenummer: 01102110.2
(22) Anmeldetag: 31.01.2001
(51) Int. Cl.: C07C 67/14, C07C 69/86, C07C 67/317, C07C 69/732, C09K 19/06

(54) **Ester aromatischer Hydroxycarbonsäuren**
Esters of aromatic hydroxycarboxylic acids
Esters d'acides aromatiques hydroxycarboxyliques

(30) Priorität: 30.03.2000 DE 10015846
(43) Veröffentlichungstag der Anmeldung: 31.10.2001
(73) Patentinhaber: Consortium für elektrochemische Industrie GmbH, 81379 München (DE)
(72) Erfinder: Sorger, Klaus, Dr., 81371 München (DE); Häberle, Norman, Dr., 80689 München (DE)
(74) Vertreter: Fritz, Helmut

(56) Entgegenhaltungen:
- EP-A- 1 059 282
- MARY E. NEUBERT ET AL.: "The Effect of Carbonyl Containing Substituents in the Terminal Chains on Mesomorphic Properties in Some Aromatic Esters and Thioesters. 3. Acyloxy Groups on the Acid End" MOLECULAR CRYSTALS AND LIQUID CRYSTALS (INC. NONLINEAR OPTICS )., Bd. 154, 1988, Seiten 127-149, XP002163711 GORDON AND BREACH SCIENCE PUBLISHERS, READING., GB
- Organikum, 16. bearb. Auflage, VEB Deutscher Verlag der Wissenschaften, Berlin, 1986, S. 233, 402
- J. March, Advanced Organic Chemistry, 4. Aufl. Verlag John Wiley & Sons, 1992, S. 392

## Beschreibung

Die Erfindung betrifft ein Verfahren zur Herstellung von Estern aromatischer Hydroxycarbonsäuren, 3-Halogenpropionsäureester aromatischer Hydroxycarbonsäuren und deren Verwendung.

Acrylsäureester aromatischer Hydroxycarbonsäuren sind wichtige polymerisierbare mesogene Bausteine, beispielsweise für flüssigkristalline Verbindungen und für Dotierstoffe für flüssigkristalline Verbindungen zur Induzierung der cholesterischen Phase.

Aus H.-J. Lorkowski, F. Reuther, Acta Chim. Acad. Sci. Hung. (1977), 95, 423-434 und M. Ueda, T. Okada, Macromolecules (1994), 27, 3449-3452 ist beispielsweise ein Verfahren zur Herstellung von 4-Acryloyloxybenzoesäure durch Umsetzung von 4-Hydroxybenzoesäure mit Acrylsäurechlorid in wäßriger alkalischer Lösung (Schotten-Baumann-Methode) bekannt.

Die genannten vorbekannten Verfahren haben folgende Nachteile: Unter den Bedingungen der genannten Veresterungsverfahren neigen aromatische Hydroxycarbonsäuren unter Bildung von Polyester-Oligomeren zur Selbstkondensation (Polykondensation). Das zugesetzte Veresterungsreagens, das Carbonsäurehalogenid, -anhydrid oder die Carbonsäure, reagiert mit der freien Hydroxylgruppe der Oligoester der aromatischen Hydroxycarbonsäure unter Bildung von acylierten Polyester-Oligomeren. Acylierte Polyester-Oligomere der Hydroxybenzoesäure werden beispielsweise durch Formel (0) wiedergegeben, wobei m den Oligomerisierungsgrad angibt:

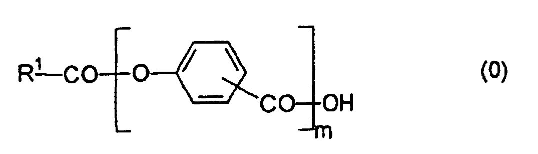

Im Reaktionsgemisch liegen deshalb neben der gewünschten veresterten aromatischen Hydroxycarbonsäure der nachstehenden allgemeinen Formel (1) acylierte Polyester-Oligomere, für Hydroxybenzoesäure durch Formel (0) wiedergegeben, als störende Verunreinigungen vor. Diese Polyester-Oligomere lassen sich nur verlustreich durch (mehrfaches) Umkristallisieren vom Zielmolekül, der veresterten aromatischen Hydroxycarbonsäure der nachstehenden allgemeinen Formel (1), entfernen.
Die Gewichtsausbeute, bezogen auf die veresterte aromatische Hydroxycarbonsäure der nachstehenden allgemeinen Formel (1), ist durch die Bildung von acylierten Polyester-Oligomeren und die Reinigung des Produkts durch (mehrfaches) Umkristallisieren notwendigerweise reduziert.
Zudem erfordert die Abtrennung der acylierten Polyester-Oligomere durch Umkristallisieren z. T. erhebliche Mengen an organischem Lösungsmittel wie z. B. Ethanol oder Essigsäure, das in der Regel verworfen werden muß.

Es bestand daher die Aufgabe, ein ökonomisches Verfahren zu entwickeln, das es erlaubt, Acrylsäureester aromatischer Hydroxycarbonsäuren in hoher Ausbeute und Reinheit herzustellen.

Gegenstand der Erfindung ist ein Verfahren zur Herstellung von Estern aromatischer Hydroxycarbonsäuren der allgemeinen Formel (1)

(R¹-COO)ₙ-Ar-X-COOH (1),

bei dem in einem ersten Schritt eine aromatische Hydroxycarbonsäure der allgemeinen Formel (2)

(HO)ₙ-Ar-X-COOH (2),

in wäßriger Lösung in Gegenwart von mit Wasser mischbarem organischem Lösungsmittel und in Gegenwart von in Wasser löslicher oder mit Wasser mischbarer Base durch 3-Chlor- oder 3-Brompropionsäurehalogenid zum Basensalz der Ester aromatischer Hydroxycarbonsäuren der allgemeinen Formel (1) verestert wird und
in einem zweiten Schritt das Basensalz der Ester aromatischer Hydroxycarbonsäuren der allgemeinen Formel (1) durch Zugabe von Säure zum freien Ester aromatischer Hydroxycarbonsäuren der allgemeinen Formel (1) umgesetzt wird,
wobei in den vorstehenden allgemeinen Formeln (1) und (2)
- **R**^{**1**}: eine 2-Chlorethyl-, eine 2-Bromethyl- oder eine Ethenylgruppe,
- **n**: eine ganze Zahl im Wert von 1, 2 oder 3
- **Ar**: ein substituiertes oder unsubstituiertes Arylen oder Heteroarylen und
- **X**: eine direkte Bindung oder eine Ethenylgruppe bedeuten.

Im erfindungsgemäßen Verfahren wird im ersten Schritt die aromatische Hydroxycarbonsäure der allgemeinen Formel (2) mit der Base zum ein-, zwei-, drei- oder gegebenenfalls vierwertigen Salz der aromatischen Hydroxycarbonsäure umsetzt. Bei vorzugsweise Temperaturen von -30 bis +30 °C wird dieses Salz in wäßriger Lösung mit 3-Halogenpropionsäurehalogenidverestert. Vorzugsweise wird zuerst die Base, gegebenenfalls danach Lösungsmittel und zuletzt 3-Halogenpropionsäurehalogenid zugegeben.
Vorzugsweise wird im zweiten Schritt der Ester aromatischer Hydroxycarbonsäuren der allgemeinen Formel (1) nach Zugabe der Säure ausgefällt und danach abgetrennt.

Zum Erläuterung des erfindungsgemäßen Verfahrens sind die Reaktionsschritte im nachfolgenden Reaktionsschema, ausgehend von 4-Hydroxybenzoesäure als Beispiel einer aromatischen Hydroxycarbonsäure der allgemeinen Formel (2) und Verwendung von 3-Chlorpropionsäurechlorid vereinfacht wiedergegeben. 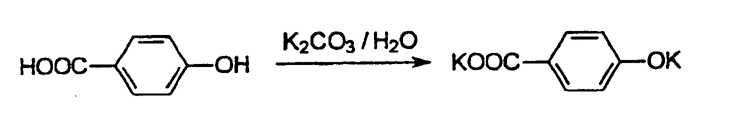
4-Hydroxybenzoesäure wird durch Verwendung von. Kaliumcarbonat als in Wasser gelöste Base zum Kaliumsalz der 4-Hydroxybenzoesäure umgesetzt.

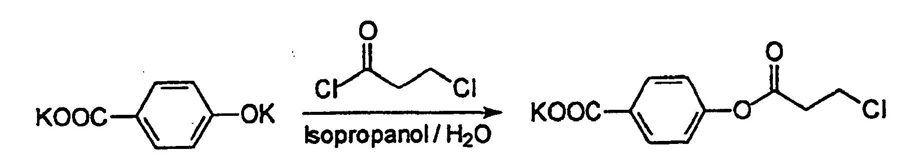

Nach Zugabe von Isopropanol als mit Wasser mischbares organisches Lösungsmittel setzt man das Kaliumsalz der 4-Hydroxybenzoesäure mit 3-Chlorpropionsäurechlorid zum Kaliumsalz der 4-(3'-Chlorpropionyloxy)benzoesäure um.

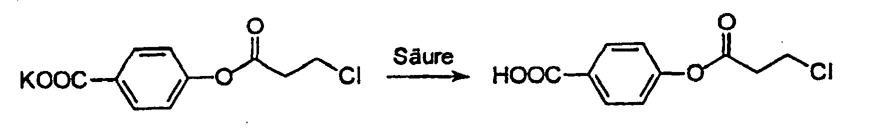

Durch Zugabe von Säure wird 4-(3'-Chlorpropionyloxy)benzoesäure ausgefällt und anschließend abgetrennt.

Es war nicht vorhersehbar, daß die Umsetzung von 3-Halogenpropionsäurehalogenid mit einer aromatischen Hydroxycarbonsäure der allgemeinen Formel (2) in wäßrigem, alkalischem Medium mit sehr hoher Ausbeute verläuft. Üblicherweise werden hydrolysebeständigere, durch Konjugation stabilisierte Säurehalogenide im wäßrigen Medium mit Alkoholen, insbesondere mit phenolischen Hydroxylgruppen, umgesetzt. Dem Fachmann sind die Bedingungen der Schotten-Baumann-Methode wohl bekannt.

Die Vorteile des erfindungsgemäßen Verfahrens gegenüber den genannten vorbekannten Verfahren bestehen darin, daß man die gewünschte veresterte aromatische Hydroxycarbonsäure der allgemeinen Formel (1) in hoher Ausbeute und Reinheit ohne Zwischenisolation und ohne Umkristallisation erhält. Insbesondere wird durch das erfindungsgemäße Verfahren die Bildung von acylierten Polyester-Oligomeren, für Hydroxybenzoesäure vorstehend durch die allgemeine Formel (0) wiedergegeben, als störende Verunreinigungen gegenüber den genannten vorbekannten Verfahren drastisch reduziert, wodurch der gewünschte Ester aromatischer Hydroxycarbonsäuren der allgemeinen Formel (1) ohne zusätzliche Umkristallisation in hoher Ausbeute erhalten wird.

Aus den 3-Halogenpropionsäureestern aromatischer Hydroxycarbonsäuren der allgemeinen Formel (1), in der **R**^{**1**} eine 2-Chlorethyl- oder eine 2-Bromethylgruppe bedeutet, entstehen leicht durch Eliminierung von Halogenwasserstoff durch die Basen die Acrylsäureester aromatischer Hydroxycarbonsäuren der allgemeinen Formel (1), in der **R**^{**1**} eine Ethenylgruppe bedeutet.

Das erfindungsgemäße Verfahren erlaubt zusätzlich die Isolierung von 3-Halogenpropionsäureestern aromatischer Hydroxycarbonsäuren der allgemeinen Formel (1), in der **R**^{**1**} eine 2-Chlorethyl- oder eine 2-Bromethylgruppe bedeutet. Dort sind die 3-Halogenpropionsäuregruppen Schutzgruppen für die leicht polymerisierbare Acrylsäuregruppe. Die 3-Halogenpropionsäureester der allgemeinen Formel (1) sind im Gegensatz zu den Acrylsäureestern gegenüber nicht erwünschter, zufälliger Polymerisation inert. Die 3-Halogenpropionsäuregruppe läßt sich vorteilhaft erst zu einem späteren Zeitpunkt, beispielsweise innerhalb einer Synthesesequenz in die polymerisierbare Acrylsäuregruppe umwandeln. Dies kann leicht durch Eliminierung von Halogenwasserstoff durch Basen erfolgen.

Unter Arylgruppe **Ar** ist ein aromatischer Rest mit vorzugsweise bis zu 30 C-Atomen, vorzugsweise Phenyl, Naphthyl, Biphenyl und Terphenyl zu verstehen. Als Substituenten werden vorteilhaft Halogen, insbesondere F, Cl, Br, C₁-C₁₀-Alkyl, C₁-C₁₀-Alkoxy und halogensubstituiertes C₁-C₁₀-Alkyl eingesetzt. Unter einer Heteroarylgruppe ist ein aromatischer Rest mit vorzugsweise bis zu 30 C-Atomen und bis zu 4 Heteroatomen aus der Gruppe O N, S, vorzugsweise Pyridyl, Pyrimidinyl, Pyridazinyl, Chinolyl, Thiophenyl, Thiadiazolyl und Oxadiazolyl zu verstehen.

Als aromatische Hydroxycarbonsäuren der allgemeinen Formel (2) sind 4-Hydroxybenzoesäure, 3-Hydroxybenzoesäure, 4'-Hydroxybiphenyl-4-carbonsäure, 6-Hydroxy-2-naphthoesäure, 5-Hydroxy-1-naphthoesäure, 3,4-Dihydroxybenzoesäure, 3,5-Dihydroxybenzoesäure und 3-(4'-Hydroxyphenyl)acrylsäure besonders bevorzugt.

Zur Umsetzung der aromatischen Hydroxycarbonsäure der allgemeinen Formel (2) in das Salz der aromatischen Hydroxycarbonsäure sind die in Wasser löslichen Basen Alkalicarbonat, Alkalihydroxid, Erdalkalicarbonat und Erdalkalihydroxid, insbesondere Natriumcarbonat, Kaliumcarbonat, Natriumhydroxid und Kaliumhydroxid besonders geeignet.
Neben den vorstehend genannten Basen sind auch mit Wasser mischbare, vorzugsweise organische Basen geeignet. Vorzugsweise sind diese Basen in jedem Verhältnis mit Wasser bei 20°C mischbar. Alkylamine und Aminoalkohole, wie Triethylamin, Diethanolamin, Triethanolamin, insbesondere Triethylamin sind zur Umwandlung in das Hydroxycarbonsäuresalz besonders geeignet.

Es hat sich bewährt, die aromatische Hydroxycarbonsäure der allgemeinen Formel (2) mit Base im Molverhältnis 1 :(2 bis 8), insbesondere 1 :(2 bis 3) für aromatische Monohydroxycarbonsäuren der allgemeinen Formel (2) mit n = 1 und 1 :(3 bis 5) für aromatische Dihydroxycarbonsäuren der allgemeinen Formel (2) mit n = 2 und 1:(4 bis 6) für aromatische Trihydroxycarbonsäuren der allgemeinen Formel (2) mit n = 3 zum Basensalz der aromatischen Hydroxycarbonsäure der allgemeinen Formel (2) umzusetzen.

Unter Basensalz der aromatischen Hydroxycarbonsäure der allgemeinen Formel (2) wirdein Monobasensatz, ein Dibasensalz, ein Tribasensalz, gegebenenfalls ein Tetrabasensalz oder ein Gemisch dieser Salze verstanden. Ein Basenkation ist dabei dem Carbonsäurerest, das andere bzw. die anderen der Phenolatgruppe bzw. den Phenolat-gruppen zuzuordnen. In vielen Fällen hat es sich bewährt, die aromatische Hydroxycarbonsäure der allgemeinen Formel (2) mit Kaliumcarbonat oder Natriumcarbonat in Wasser zum Kaliumsalz bzw. Natriumsalz umzusetzen. Besonders einfach gestaltet sich das Verfahren, wenn man eine wäßrige Lösung des Alkalimetallcarbonats verwendet.

Man kann auch zuerst die aromatische Hydroxycarbonsäure der allgemeinen Formel (2) mit äquimolaren Mengen Alkalimetallhydroxid oder Amin zum Monoalkalimetallsalz bzw. Monoammoniumsalz umsetzen, wobei die Carboxylgruppe deprotoniert wird. Das Monoalkalimetallsalz bzw. Monoammoniumsalz wird anschließend zur Deprotonierung der aromatischen Hydroxylgruppe mit Alkalimetallcarbonat im Molverhältnis 1:(1 bis 5), insbesondere 1:(1 bis 2) für Monohydroxycarbonsäuren der allgemeinen Formel (2) mit n = 1, 1 :(2 bis 3) für Dihydroxycarbonsäuren der allgemeinen Formel (2) mit n = 2 und 1 :(3 bis 4) für Trihydroxycarbonsäuren der allgemeinen Formel (2) mit n = 3 zum höherwertigen Alkalimetallsalz bzw. zum höherwertigen gemischten Alkalimetall-Ammoniumsalz der aromatischen Hydroxycarbonsäure der allgemeinen Formel (2) umgesetzt.

Man kann auch kommerziell erhältliches Monoalkalimetallsalz der aromatischen Hydroxycarbonsäure der allgemeinen Formel (2) einsetzen, das anschließend bevorzugt mit Alkalimetallcarbonat zum Dialkalimetallsalz, Trialkalimetallsalz oder gegebenenfalls höherwertigen Alkalimetallsalz umgesetzt wird.

Insbesondere gibt man zur wäßrigen Lösung des Salzes der aromatischen Hydroxycarbonsäure der allgemeinen Formel (2) ein mit Wasser mischbares organisches Lösungsmittel oder ein Gemisch mehrerer mit Wasser mischbarer organischer Lösungsmittel. Vorzugsweise sind die organischen Lösungsmittel in jedem Verhältnis mit Wasser bei 20°C mischbar. Als organische, mit Wasser mischbare Lösungsmittel sind Tetrahydrofuran, Acetonitril, N,N-Dimethylformamid, N,N-Dimethylacetamid, Dimethylsulfoxid und N-Methyl-2-pyrrolidinon, 1,3-Dimethyl-2-imidazolidinon und insbesondere Methanol, Ethanol, Isopropanol und Aceton besonders geeignet.

Es hat sich bewährt, zur wäßrigen Lösung des Salzes der aromatischen Hydroxycarbonsäure der allgemeinen Formel (2) das mit Wasser mischbare organische Lösungsmittel in einem Volumenverhältnis Wasser: organisches Lösungsmittel von 1 :(0.01 bis 3), insbesondere 1:(0.1 bis 1) zuzusetzen. Man setzt das Basensalz der aromatischen Hydroxycarbonsäure der allgemeinen Formel (2) in wäßriger Lösung in Gegenwart eines mit Wasser mischbaren organischen Lösungsmittels, wie zuvor bereits erwähnt, bei -30 bis +30 °C, insbesondere bei -20 bis +10 °C, bevorzugt bei -15 bis +5 °C mit 3-Halogenpropionsäurehalogenid zum Monosalz des 3-Halogenpropionsäureesters der aromatischen Hydroxycarbonsäure der allgemeinen Formel (1) um.

Als 3-Halogenpropionsäurehalogenid haben sich 3-Brompropionsäurechlorid und 3-Chlorpropionsäurechlorid, bevorzugt 3-Chlorpropionsäurechlorid besonders bewährt. Man kann das Säurehalogenid in konzentrierter Form oder in Form einer verdünnten Lösung in einem mit Wasser mischbaren inerten Lösungsmittel einsetzen.

Während der Umsetzung hält man vorzugsweise die Temperatur durch Kühlen auf dem vorgegebenen Wert. Nach Ende der Zugabe von 3-Halogenpropionsäurehalogenid läßt man noch eine gewisse Zeit nachreagieren, um die Umsetzung zu vervollständigen.

Nach erfolgter Umsetzung des ersten Schritts fällt man die Carbonsäure der allgemeinen Formel (1) durch Zugabe von Säure zu dem das Monoalkalimetallsalz der aromatischen Carbonsäure der Formel (1) enthaltenden alkalischen Reaktionsgemisch bei - 30 bis +30 °C, insbesondere bei -20 bis +10 °C, bevorzugt bei -15 bis 0 °C aus. Im allgemeinen genügt es, die aromatische Carbonsäure der Formel (1) bei einem pH-Wert von 1 bis 5, insbesondere von 2 bis 4 auszufällen, dadurch, daß man dem Reaktionsgemisch soviel Säure zusetzt, bis der gewünschte, vorstehend genannte pH-Wert erreicht ist. Alternativ dazu kann man auch das das Monoalkalimetallsalz der aromatischen Carbonsäure der allgemeinen Formel (1) enthaltende alkalische Reaktionsgemisch zu einer Mineralsäure zusetzen (inverse Neutralisation).

Als Säure wird bevorzugt eine Mineralsäure verwendet. Man setzt insbesondere Salzsäure, Schwefelsäure oder Phosphorsäure, bevorzugt Salzsäure als Säure ein. Die Säure kann in konzentrierter Form oder in Form einer verdünnten wäßrigen Lösung eingesetzt werden.

Der die Ester der allgemeinen Formel (1) enthaltende Niederschlag kann durch Filtration oder Zentrifugation abgetrennt, mit Wasser gewaschen und getrocknet werden. Durch Ausfällung der Ester der allgemeinen Formel (1) durch Zugabe von Säure gestaltet sich das erfindungsgemäße Verfahren besonders einfach, da der die Ester der allgemeinen Formel (1) enthaltende Niederschlag durch Filtration oder Zentrifugation, insbesondere Filtration unmittelbar abgetrennt werden kann, ohne daß ein mit Wasser nicht mischbares organisches Lösungsmittel zugefügt wird, in dem sich das Monosalz löst. Aus diesem Grund wird üblicherwiese dieser vorteilhaften Verfahrensvariante der Vorzug gegeben.

Das erfindungsgemäße Verfahren gestaltet sich besonders einfach, da auf eine zusätzliche Reinigung der erfindungsgemäß hergestellten Ester aromatischer Hydroxycarbonsäuren der allgemeinen Formel (1) verzichtet werden kann.

Nach dem erfindungsgemäßen Verfahren lassen sich 3-Halogenpropionsäureester aromatischer Hydroxycarbonsäuren der allgemeinen Formel (1), in sehr hohen Ausbeuten von bis zu > 90 % gewinnen. Üblicherweise beträgt der Anteil an acylierten Polyester-Oligomeren, für Hydroxybenzoesäure durch Formel (0) wiedergegeben, als störende Verunreinigungen weniger als 2 %. Der Anteil von durch Eliminierung von Halogenwasserstoff gebildetem Acrylsäureester der allgemeinen Formel (1), wobei **R**^{**1**} eine Ethenylgruppe bedeutet, beträgt in den meisten Fällen weniger als 3%.

Man kann das erfindungsgemäße Verfahren auch so gestalten, daß ausschließlich der Acrylsäureester der aromatischen Hydroxycarbonsäure der allgemeinen Formel (1), wobei **R**^{**1**} eine Ethenylgruppe bedeutet, gewonnen wird. Üblicherweise erwärmt man dazu nach erfolgter Umsetzung das das Monobasensalz der aromatischen Carbonsäure der allgemeinen Formel (1) enthaltende alkalische Reaktionsgemisch, wobei **R**^{**1**} eine 2-Chlor- oder eine 2-Bromethylgruppe bedeutet, auf 20 bis 90 °C, bevorzugt auf 30 bis 50 °C. Im alkalischen Medium wird dabei die 2-Chlorethylgruppe oder die 2-Bromethylgruppe durch Eliminierung von Halogenwasserstoff zur Ethenylgruppe umgesetzt. Nach Beendigung der Umsetzung kühlt man das Reaktionsgemisch vorzugsweise auf -15 bis +40 °C, insbesondere 0 bis 20 °C, ab und fällt die freie Carbonsäure der allgemeinen Formel (1), wobei **R**^{**1**} eine Ethenylgruppe bedeutet, wie zuvor bereits erwähnt, durch Zugabe von Säure aus.

Man kann auch nach erfolgter Umsetzung das das Monoalkalimetallsalz der aromatischen Carbonsäure der allgemeinen Formel (1) enthaltende alkalische Reaktionsgemisch, wobei **R**^{**1**} eine 2-Chlor- oder eine 2-Bromethylgruppe bedeutet, mit zusätzlichen Anteilen der Base versetzen. Durch die überschüssige Base wird dabei im alkalischen Medium die 2-Chlorethylgruppe oder die 2-Bromethylgruppe zur Ethenylgruppe umgesetzt. Als mit Wasser mischbare Base ist dafür eine der vorstehend beschriebenen Amine, insbesondere Triethylamin besonders geeignet. Nach Beendigung der Umsetzung fällt man die Carbonsäure der allgemeinen Formel (1), wobei **R**^{**1**} eine Ethenylgruppe bedeutet, wie zuvor bereits erwähnt, durch Zugabe von Säure aus.

Altemativ kann man die isolierte, vorzugsweise durch Filtration abgetrennte, gewaschene und getrocknete Säure der allgemeinen Formel (1), wobei **R**^{**1**} eine 2-Chlor- oder eine 2-Bromethylgruppe bedeutet, auch in Gegenwart von Acrylsäureester der allgemeinen Formel (1), wobei **R**^{**1**} eine Ethenylgruppe bedeutet, in einem organischen Lösungsmittel, insbesondere Methyl-tert.-butylether, Methylethylketon, Diethylketon, Ethylacetat oder Butylacetat auflösen, mit einer Base oder einem Gemisch mehrerer Basen im Molverhältnis 1:(1 bis 8), bevorzugt 1 :(1 bis 4) versetzen und auf 30 bis 100°C, insbesondere 30 bis 60 °C erwärmen und damit durch Eliminierung von Halogenwasserstoff den 3-Halogenpropionsäureester zum Acrylsäureester der allgemeinen Formel (1), wobei **R**^{**1**} eine Ethenylgruppe bedeutet, umsetzen.

Das erfindungsgemäße Verfahren gestaltet sich besonders einfach, da gegenüber den vorbekannten Verfahren auf eine zusätzliche Reinigung der veresterten aromatischen Hydroxycarbonsäure der allgemeinen Formel (1), wobei R¹ eine Ethenylgruppe bedeutet, durch Umkristallisation verzichtet werden kann.

Nach dem vorstehend beschriebenen, erfindungsgemäßen Verfahren lassen sich damit auch Acrylsäureester aromatischer Hydroxycarbonsäuren der allgemeinen Formel (1), wobei **R**^{**1**} eine Ethenylgruppe bedeutet, in sehr hohen Ausbeuten von bis zu > 90 % gewinnen. Üblicherweise beträgt der Anteil an acylierten Polyester-Oligomeren, für Hydroxybenzoesäure durch Formel (0) wiedergegeben, als störende Verunreinigungen weniger als 2 %.

Die nach dem erfindungsgemäßen Verfahren hergestellten 3-Halogenpropionsäureester aromatischer Hydroxycarbonsäuren der allgemeinen Formel (1), sind bei geeigneter Bauweise auch mesogene Bausteine und werden zur Herstellung von flüssigkristallinen polymerisierbaren Verbindungen und zur Herstellung von polymerisierbaren Dotierstoffen für flüssigkristalline Verbindungen zur Induzierung der cholesterischen Phase verwendet. Die Herstellung der flüssigkristallinen Verbindungen und der Dotierstoffe fürflüssigkristalline Verbindungen unter Verwendung der nach dem erfindungsgemäßen Verfahren hergestellten 3-Halogenpropionsäureester aromatischer Hydroxycarbonsäuren der allgemeinen Formel (1) erfolgt nach an sich bekannten Methoden.

Die 3-Halogenpropionsäuregruppe wird dabei in Gegenwart von Basen durch Eliminierung von Halogenwasserstoff zur polymerisierbaren Acrylsäuregruppe umgesetzt. Man kann auch nach dem erfindungsgemäßen Verfahren hergestellte Gemische eines 3-Halogenpropionsäure- und Acrylsäureesters einer aromatischen Hydroxycarbonsäure der allgemeinen Formel (1), wobei **R**^{**1**} eine 2-Chlorethyl- oder eine 2-Bromethylgruppe und eine Ethenylgruppe bedeutet, zur Herstellung der flüssigkristallinen polymerisierbaren Verbindungen und der polymerisierbaren Dotierstoffe für flüssigkristalline Verbindungen einsetzen. Einzelheiten zur Herstellung können den nachstehend genannten Beispielen entnommen werden.

Das erfindungsgemäße Verfahren ist neben den vorstehend genannten 3-Halogenpropionsäure- und Acrylsäureestern der allgemeinen Formel (1) generell zur Herstellung von Estern aromatischer Hydroxycarbonsäuren der allgemeinen Formel (1) geeignet, wobei **R**^{**1**} einen organischen Rest bezeichnet, insbesondere eine lineare oder verzweigte oder cyclische C₁-C₁₅-Alkyl-, C₁-C₁₅-Alkoxyalkyl-, halogensubstituierte C₂-C₁₅-Alkyl-, substituierte oder unsubstituierte Aryl-C₁-C₁₅-Alkyl-, substituierte oder unsubstituierte Heteroaryl-C₁-C₁₅-Alkyl-, substituierte oder unsubstituierte Alkenyl-, substituierte oder unsubstituierte Aryl-, und substituierte oder unsubstituierte Heteroarylgruppe bedeutet. Die nach dem vorstehend beschriebenen, erfindungsgemäßen Verfahren hergestellten Verbindungen werden in höheren Ausbeuten erhalten als in bekannten Verfahren.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1: Herstellung von 4-(3'-Chlorpropionyloxy)benzoesäure

Bei Raumtemperatur wurden in einer Lösung von 12,5 g Kaliumcarbonat (90,5 mmol) in 45 ml Wasser 5 g 4-Hydroxybenzoesäure (36,2 mmol) aufgelöst. Nachdem zu dieser Lösung 15 ml Isopropanol zugefügt wurden, kühlte man die Mischung auf -15°C und tropfte 5,5 g 3-Chlorpropionsäurechlorid (43,5 mmol) unverdünnt innerhalb 10 min zu, wobei die Temperatur durch externe Kühlung bei -15°C gehalten wurde. Dann wurde die Mischung 30 min bei - 10°C gerührt, mit 24 ml 6 N Salzsäure bis zu einem pH-Wert von 2 angesäuert, wobei die Temperatur auf 0 °C anstieg und das Produkt in Form eines weißen Niederschlags ausfiel. Dieser Niederschlag wurde abfiltriert, dreimal mit je 10 ml Wasser gewaschen und getrocknet. 4-(3'-Chlorpropionyloxy)benzoesäure wurde in einer Ausbeute von 8 g (97 % d. Th.) mit einem Schmp. von 177°C erhalten. Die Verbindung enthält 3,1 Mol% 4-Acryloyloxybenzoesäure und 0,8 Mol-% acylierte oligomere Verbindungen.

### Beispiel 2: Herstellung eines Gemisches aus 4-(3'-Chlorpropionyloxy)benzoesäure und 4-Acryloyloxybenzoesäure

Bei Raumtemperatur wurden in einer Lösung von 9,74 g Kaliumhydroxid (148 mmol) in 87 ml Wasser 10 g 4-Hydroxybenzoesäure (72 mmol) aufgelöst. Nachdem zu dieser Lösung 30 ml lsopropanol zugefügt wurden, kühlte man die Mischung auf -15 °C und tropfte 10,1 g 3-Chlorpropionsäurechlorid (80 mmol) unverdünnt innerhalb 10 min zu, wobei die Temperatur auf -10°C anstieg. Dann wurde die Mischung 20 min bei -5 °C gerührt, mit 50 ml Methylethylketon versetzt und anschließend zu 17 ml 6 N Salzsäure getropft, wobei die Temperatur auf +5 °C anstieg. Die Mischung wurde dann auf 30 °C erwärmt, wobei nicht gelöste Bestandteile in Lösung gingen. Die organische Phase wurde viermal mit je 40 ml Wasser gewaschen, über Natriumsulfat getrocknet und das Lösungsmittel im Vakuum entfernt. 4-(3'-Chlorpropionyloxy)-benzoesäure wurde in einer Ausbeute von 14,5 g (88 % d. Th.) mit einem Schmp. von 178 °C erhalten. Die Verbindung enthält 8 % 4-Acryloyloxybenzoesäure und 0,6 Mol-% acylierte oligomere Verbindungen.

### Beispiel 3: Herstellung von 3-(3'-Chlorpropionyloxy)benzoesäure

Bei Raumtemperatur wurden in einer Lösung von 20 g Kalium carbonat (145 mmol) in 90 ml Wasser 10 g 3-Hydroxybenzoesäure (72,4 mmol) aufgelöst. Nachdem zu dieser Lösung 30 ml Isopropanol zugefügt wurden, kühlte man die Mischung auf -15 °C und tropfte 11,1 g 3-Chlorpropionsäurechlorid (87 mmol) unverdünnt innerhalb 10 min zu, wobei die Temperatur auf -10 °C anstieg. Dann wurde die Mischung 30 min bei -10 °C gerührt, mit 50 ml Methylethylketon versetzt und mit 30 ml 6 N Salzsäure bis zu einem pH-Wert von 1 angesäuert, wobei die Temperatur auf 0 °C anstieg. Die organische Phase wurde mit 30 ml Wasser gewaschen und das Lösungsmittel im Vakuum entfernt. Der erhaltene Feststoff wurde zur Entfernung von 3-Chlorpropionsäureresten in 50 ml Wasser suspendiert und über Nacht bei Raumtemperaturgerührt. Nach Filtration und Trocknung wurde 3-(3'-Chlorpropionyloxy)benzoesäure in einer Ausbeute von 14,5 g (88 % d. Th.) mit einem Schmp. von 98 °C erhalten. Die Verbindung enthält 2,5 % 3-Acryloyloxybenzoesäure (acylierte oligomere Verbindungen sind nicht nachweisbar).

### Beispiel 4: Herstellung von 6-(3'-Chlorpropionyloxy)-2-naphthoesäure

Bei Raumtemperatur wurden in einer Lösung von 30 g Kaliumcarbonat (217 mmol) in 135 ml Wasser 20,4 g 6-Hydroxy-2-naphthoesäure (109 mmol) aufgelöst. Nachdem zu dieser Lösung 45 ml Isopropanol zugefügt wurden, kühlte man die Mischung auf 15 °C und tropfte 16,6 g 3-Chlorpropionsäurechlorid (130,4 mmol) unverdünnt innerhalb 10 min zu. Dann wurde die Mischung 15 min bei 15 °C gerührt und mit 47 ml 6 N Salzsäure bis zu einem pH-Wert von 2 angesäuert, wobei das Produkt in Form eines weißen Niederschlags ausfiel. Dieser Niederschlag wurde abfiltriert, dreimal mit je 150 ml Wasser gewaschen und getrocknet. 6-(3'-Chlorpropionyloxy)-2-naphthoesäure wurde in einer Ausbeute von 28,3 g (95 % d. Th.) mit einem Schmp. von 210°C erhalten. Die Verbindung enthält 12,7 % 6-Acryloyloxy-2-naphthoesäure und 2,1 % acylierte oligomere Verbindungen.

### Beispiel 5: Herstellung von 3,5-Di-(3'-chlorpropionyloxy)-benzoesäure

Bei Raumtemperatur wurden in einer Lösung von 28,8 g Kaliumcarbonat (208 mmol) in 90 ml Wasser 10 g 3,5-Dihydroxybenzoesäure (65 mmol) aufgelöst. Nachdem zu dieser Lösung 30 ml Isopropanol zugefügtwurden, kühlte man die Mischung auf -15 °C und tropfte 19,2 g 3-Chlorpropionsäurechlorid (150 mmol) unverdünnt innerhalb 20 min zu, wobei die Temperatur auf -10 °C anstieg. Dann wurde die Mischung 30 min bei -10 °C gerührt, mit 70 ml Methylethylketon versetzt, mit 32 ml 6 N Salzsäure bis zu einem pH-Wert von 1 angesäuert und auf 35 °C erwärmt, wobei sich eine klare Lösung bildete. Die organische Phase wurde mit 30 ml Wasser gewaschen und das Lösungsmittel im Vakuum entfernt. Der erhaltene Feststoff wurde zur Entfernung von 3-Chlorpropionsäureresten in 150 ml *tert.-*Butylmethylether gelöst, die organische Phase bei 40 °C viermal mit je 50 ml Wasser gewaschen und das Lösungsmittel im Vakuum entfernt. 3,5-Di-(3'-chlorpropionyloxy)benzoesäure wurde in einer Ausbeute von 18,8 g (86 % d. Th.) mit einem Schmp. von 138 °C erhalten. Die Verbindung enthält3,1 % des Monoacrylsäureesterderivats und 2,3 % acylierte oligomere Verbindungen.

### Beispiel 6: Herstellung von 4-Acryloyloxybenzoesäure

Bei Raumtemperatur wurden in einer Lösung von 30 g Kaliumcarbonat (217 mmol) in 90 ml Wasser 10 g 4-Hydroxybenzoesäure (72,4 mmol) aufgelöst. Nachdem zu dieser Lösung 30 ml Isopropanol zugefügt wurden, kühlte man die Mischung auf -15 °C und tropfte 11,1 g 3-Chlorpropionsäurechlorid (87 mmol) unverdünnt innerhalb 10 min zu, wobei die Temperatur durch exteme Kühlung bei -15 °C gehalten wurde. Die Mischung wurde 30 min bei -10 °C gerührt, dann auf 40 °C erwärmt, 90 min bei dieser Temperatur gerührt, auf 15 °C abgekühlt und mit 30 ml 6 N Salzsäure bis zu einem pH-Wert von 2 angesäuert, wobei das Produkt in Form eines weißen Niederschlags ausfiel. Dieser Niederschlag wurde abfiltriert und dreimal mit je 50 ml Wasser gewaschen. Der erhaltene Feststoff wurde zur Entfernung von 3-Chlorpropionsäureresten in 100 ml Wasser suspendiert und 30 min bei 30 °C gerührt. Nach Filtration undTrocknung wurde 4-Acryloyloxybenzoesäure in einer Ausbeute von 10,6 g (76 % d. Th.) erhalten (der Schmp. konnte nicht bestimmt werden, da die Verbindung polymerisiert, ohne vorher geschmolzen zu sein). Die Verbindung enthält 5 % 4-(3'-Chlorpropionyloxy)benzoesäure und 0.6 % 4-Hydroxybenzoesäure (acylierte oligomere Verbindungen sind nicht nachweisbar).

### Beispiel 7: Herstellung von 4-Octanoyloxybenzoesäure

Bei Raumtemperatur wurden in einer Lösung von 20 g Kaliumcarbonat (145 mmol) in 90 ml Wasser 10 g 4-Hydroxybenzoesäure (72,4 mmol) aufgelöst. Nachdem zu dieser Lösung 30 ml lsopropanol zugefügt wurden, kühlte man die Mischung auf -15 °C und tropfte 15,3 g Octansäurechlorid (94 mmol) unverdünnt innerhalb 20 min zu, wobei die Temperatur auf -10 °C anstieg. Dann wurde die Mischung 30 min bei -10 °C gerührt, mit 50 ml Methylethylketon versetzt, mit 28 ml 6 N Salzsäure bis zu einem pH-Wert von 2 angesäuert und auf 35 °C erwärmt, wobei sich eine klare Lösung bildete. Die organische Phase wurde mit 50 ml auf 35 °C erwärmten Wasser gewaschen und das Lösungsmittel im Vakuum entfernt. Der erhaltene Feststoff wurde zur Entfernung von Octansäureresten in 50 ml Petrolether suspendiert und 30 min bei RT gerührt. Nach Filtration und Trocknung wurde 4-Octanoyloxybenzoesäure in einer Ausbeute von 15,4 g (80 % d. Th.) mit einem Schmp. von 146 °C erhalten (acylierte oligomere Verbindungen sind nicht nachweisbar).

### Beispiel 8: Herstellung von 4-Acetoxybenzoesäure

Bei Raumtemperatur wurden in einer Lösung von 20 g Kaliumcarbonat (145 mmol) in 90 ml Wasser 10 g 4-Hydroxybenzoesäure (72,4 mmol) aufgelöst. Nachdem zu dieser Lösung 30 ml Isopropanol zugefügt wurden, kühlte man die Mischung auf -15 °C und tropfte 11,4 g Essigsäurechlorid (145 mmol) unverdünnt innerhalb 15 min zu, wobei die Temperatur auf -10 °C anstieg. Dann wurde die Mischung 20 min bei -10 °C gerührt, mit 50 ml Methylethylketon versetzt, mit 35 ml 6 N Salzsäure bis zu einem pH-Wert von 2 angesäuert und auf 25 °C erwärmt, wobei sich eine klare Lösung bildete. Nachdem zur Phasentrennung 20 ml einer gesättigten Natriumchloridlösung zugefügt wurden, wurde die organische Phase dreimal mit je 40 ml Wasser gewaschen, über Natriumsulfat getrocknet und das Lösungsmittel im Vakuum entfernt. 4-Acetoxybenzoesäure wurde in einer Ausbeute von 13 g (90 % d. Th.) mit einem Schmp. von 146 °C erhalten (acylierte oligomere Verbindungen sind nicht nachweisbar).

### Beispiel 9: Herstellung von 4-(2',2'-Dimethylpropionyloxy)-benzoesäure

Analog zu Beispiel 7 wurde 4-(2',2'-Dimethylpropionyloxy)benzoesäure, erhalten durch Veresterung von 4-Hydroxybenzoesäure mit 2,2-Dimethylpropionsäurechlorid, in einer Ausbeute von 84 % d. Th. mit einem Schmp. von 185 °C hergestellt. Die Verbindung enthält 3,1 % acylierte oligomere Verbindungen.

### Beispiel 10: Herstellung von trans-3-[4'-(4"-Chlorbutyryloxy)-phenyl]acrylsäure

Analog zu Beispiel 7 wurde trans-3-[4'-(4"-Chlorbutyryloxy)-phenyl]acrylsäure, erhalten durch Veresterung von trans-3-(4'-Hydroxyphenyl)acrylsäure mit 4-Chlorbuttersäurechlorid, in einer Ausbeute von 93 % d. Th. mit einem Schmp. von 160-161 °C hergestellt. Die Verbindung enthält 1,6 % acylierte oligomere Verbindungen.

### Beispiel 11: Herstellung von 4-(4'-Methylbenzoyloxy)benzoesäure

Bei Raumtemperatur wurden in einer Lösung von 20 g Kaliumcarbonat (145 mmol) in 90 ml Wasser 10 g 4-Hydroxybenzoesäure (72,4 mmol) aufgelöst. Nachdem zu dieser Lösung 30 ml lsopropanol zugefügt wurden, kühlte man die Mischung auf -12 °C und tropfte 11,4 g 4-Methylbenzoylchlorid (74 mmol) unverdünnt innerhalb 10 min zu. Die Mischung wurde 30 min bei -5 °C gerührt und mit 32 ml 6 N Salzsäure bis zu einem pH-Wert von 2 angesäuert, wobei das Produkt in Form eines weißen Niederschlags ausfiel. Dieser Niederschlag wurde abfiltriert und dreimal mit je 50 ml Wasser gewaschen. Der erhaltene Feststoff wurde zur Entfernung von 3-Methylbenzoesäureresten in 200 ml Wasser suspendiert und 30 min bei 65 °C gerührt. Nach Filtration und Trocknung wurde 4-(4'-Methylbenzoyloxy)benzoesäure in einer Ausbeute von 16,3 g (88 % d. Th.) mit einem Schmp. von 239 °C erhalten. Die Verbindung enthält 0,3 % 3-Methylbenzoesäure und 0,7 % acylierte oligomere Verbindungen.

### Beispiel 12: Herstellung des chiralen Dotierstoffes für flüssigkristalline Verbindungen Di-2,5-[(6'-acryloyloxy)-2'naphthoyl]isosorbid

Zur Suspension von 19,7 g (71,7 mmol) des in Beispiel 3 hergestellten Gemisches aus 6-(3'-Chlorpropionylo-xy)-2-naphthoesäure und 6-Acryloyloxy-2-naphthoesäure in 60 ml Toluol wurden bei 70 °C 17,1 g Thionylchlorid (143.4 mmol) innerhalb 10 min getropft. Die Mischung wurde anschließend auf 90 °C erwärmt und 5 h gerührt, wobei sich eine klare Lösung bildete. Nach Abkühlung auf Raumtemperatur wurde das Lösungsmittel im Vakuum bis auf einen Toluolgehalt von etwa 50 Gew.-% entfernt. Die erhaltene Lösung von 6-(3'-Chlorpropionyloxy)-2-naphthoesäurechlorid wurde ohne weitere Reinigung eingesetzt. 5 g Isosorbid (34,1 mmol) wurden in 35 ml Toluol suspendiert. Zu dieser Mischung wurden 500 ppm 2,6-Di-tert.-butyl-4-(dimethylaminomethylen)-phenol (Ethanox® 703, Ethyl Corp., Baton Rouge, LA 70801) als Stabilisator zugefügt. Die Mischung wurde auf 65 °C erwärmt, wobei sich eine klare Lösung bildete. Zu dieser Lösung wurden 17,3 g Triethylamin (170,5 mmol) zugefügt und anschließend 21 g 6-(3'-Chlorpropionyloxy)-2-naphthoesäurechlorid (71,7 mmol), gelöst in 25 ml Toluol, zugetropft. Die Mischung wurde 5 h bei 80°C gerührt, mit 280 ml Methylethylketoh und 100 ml 1 N Salzsäure versetzt. Die organische Phase wurde mit 100 ml Wasser und 100 ml 5 %-iger Natriumhydrogencarbonat-Lösung gewaschen, über Natriumsulfat getrocknet und das Lösungsmittel im Vakuum entfernt. Der erhaltene Feststoff wurde mit 300 ml Methylethylketon versetzt und unter Rühren auf 80 °C erwärmt. Nach Abkühlung und Filtration wurde das Di-2,5-[(6'-acryloyloxy)-2'-naphthoyl]-isosorbid in einer Ausbeute von 9 g (44 % d. Th.) mit einem Schmp. von 183°C erhalten.

### Beispiel 13: Herstellung des chiralen Dotierstoffes für flüssigkristalline Verbindungen Di-2,5-[(4'-acryloyloxy)-benzoyl]isomannid bzw. -isosorbid

a) Zur Suspension von 75 g (328 mmol) der in Beispiel 1 hergestellten Verbindung 4-(3'-Chlorpropionyloxy)benzoesäure in 200 ml Toluol wurden bei 70 °C 58,5 g Thionylchlorid (492 mmol) innerhalb 30 min getropft. Die Mischung wurde anschließend auf 90 °C erwärmt und 2 h gerührt, wobei sich eine klare Lösung bildete. Nach Abkühlung auf Raumtemperatur wurde das Lösungsmittel im Vakuum bis auf einen Toluolgehalt von etwa 50 Gew.-% entfernt. Die erhaltene Lösung von 4-(3'-Chlorpropionyloxy)benzoesäurechlorid wurde ohne weitere Reinigung eingesetzt.
b) 22,9 g lsomannid (156 mmol) wurden in 300 ml Toluol suspendiert. Zu dieser Mischung wurden 1000 ppm 2,6-Di-tert.-butyl-4-(dimethylaminomethylen)-phenol (Ethanox® 703, Ethyl Corp., Baton Rouge, LA 70801) als Stabilisator zugefügt. Die Mischung wurde auf 70 °C erwärmt, wobei sich eine klare Lösung bildete. Zu dieser Lösung wurden 100 g Triethylamin (720 mmol) zugefügt und anschließend 81 g 4-(3'-Chlorpropionyloxy)benzoesäurechlorid (328 mmol), gelöst in 90 ml Toluol, zugetropft. Die Mischung wurde 2 h bei 80 °C gerührt und anschließend mit 60 ml Wasser versetzt. Die organische Phase wurde bei 60 °C mit 60 ml 10 %-iger Salzsäure versetzt, mit 60 ml Wasser, 60 ml 10 %-iger Natriumhydrogencarbonat-Lösung und 60 ml Wasser gewaschen und über Natriumsulfat getrocknet. Die erhaltene Lösung wurde mit 300 ml Toluol versetzt und über Tonsil® 411 filtriert. Dann wurden ca. 500 ml Toluol im Vakuum abdestilliert. Der Rückstand wurde mit 200 ml Ethanol und 350 ml Cyclohexan versetzt und auf 80 °C erwärmt, wobei sich eine klare Lösung bildete. Nach Abkühlung und Filtration wurde das Di-2,5-[(4'-acryloyloxy)-benzoyl]isomannid in einer Ausbeute von 58 g (75 % d. Th.) mit einem Schmp. von 133°C erhalten. c) In analoger Weise wurde durch Umsetzung von Isosorbid mit 4-(3'-Chlor-propionyloxy)benzoesäurechlorid Di-2,5-[(4'-acryloyloxy)benzoyl]isosorbid mit einem Schmp. von 114-115 °C erhalten.

### Beispiel 14: Herstellung der flüssigkristallinen Verbindung 4-Acryloyloxybenzoyl-4'-cyanophenylester

Bei 40 °C wurden 31,6 g 4-(3'-Chlorpropionyloxy)benzoesäurechlorid (128 mmol), hergestellt in Beispiel 13a, und 14,3 g 4-Cyanophenol (120 mmol) in 110 ml Toluol gelöst. Zu dieser Mischung wurden 1000 ppm 2,6-Di-tert.-butyl-4-(dimethylaminomethylen)phenol (Ethanox® 703, Ethyl Corp., Baton Rouge, LA 70801) als Stabilisator zugefügt und anschließend 30,3 g Triethylamin (300 mmol) zugetropft. Die Mischung wurde auf 60 °C erwärmt und 3,5 h bei dieser Temperatur gerührt. Nach Abkühlung auf 40 °C wurde mit 200 ml Ethylacetat und 100 ml Wasser versetzt, die organische Phase zweimal mit je 50 ml 5 %-iger Salzsäure, 50 ml 5 %-iger Natriumhydrogencarbonat-Lösung und 50 ml Wasser gewaschen und über Natriumsulfat getrocknet. Das Lösungsmittel wurde im Vakuum abdestilliert und der Rückstand aus 220 ml Isopropanol umkristallisiert. Der erhaltene Feststoff wurde abfiltriert, mit 100 ml kaltem lsopropanol gewaschen und getrocknet. 4-Acryloyloxybenzoyl-4'-cyanophenylesterwurde in einer Ausbeute von 34 g (96 % d. Th.) mit einem Schmp. von 130 °C (Lit.: 134°C) erhalten. Mittels DSC (differential scanning calorimetry) konnte eine flüssigkristalline Phase zw. 130°C und 164 °C nachgewiesen werden.

## Patentansprüche

1. Verfahren zur Herstellung von Estern aromatischer Hydroxycarbonsäuren der allgemeinen Formel (1)
(R¹-COO)ₙAr-X-COOH (1),
bei dem in einem ersten Schritt eine aromatische Hydroxycarbonsäure der allgemeinen Formel (2)
(HO)ₙ-Ar-X-COOH (2),
in wäßriger Lösung in Gegenwart von mit Wasser mischbarem organischem Lösungsmittel und in Gegenwart von in Wasser löslicher oder mit Wasser mischbarer Base durch 3-Chlor- oder 3- Brompropionsäurehalogenid zum Basensalz der Ester aromatischer Hydroxycarbonsäuren der allgemeinen Formel (1) verestert wird und
in einem zweiten Schritt das Basensalz der Ester aromatischer Hydroxycarbonsäuren der allgemeinen Formel (1) durch Zugabe von Säure zum freien Ester aromatischer Hydroxycarbonsäuren der allgemeinen Formel (1) umgesetzt wird,
wobei in den vorstehenden allgemeinen Formeln (1) und (2)
**R**^{**1**} eine 2-Chlorethyl-, eine 2-Bromethyl- oder eine Ethenylgruppe,
**n** eine ganze Zahl im Wert von 1, 2 oder 3
**Ar** ein substituiertes oder unsubstituiertes Arylen oder Heteroarylen und
**X** eine direkte Bindung oder eine Ethenylgruppe bedeuten.

2. Verfahren nach Anspruch 1 oder 2, bei dem die in Wasser lösliche Base aus Alkalicarbonat, Alkalihydroxid, Erdalkalicarbonat, Erdalkalihydroxid und mit Wasser mischbaren Alkylaminen und Aminoalkoholen ausgewählt wird.

3. Verfahren nach Anspruch 2, bei dem das mit Wasser mischbare Lösungsmittel ausgewählt wird aus Methanol, Ethanol, Isopropanol und Aceton.

4. Verfahren nach Anspruch 1 bis 3, bei dem als Säure eine Mineralsäure verwendet wird.

5. 3-Halogenpropionsäureester aromatischer Hydroxycarbonsäuren der allgemeinen Formel (1)
(R¹-COO)ₙ-Ar-X-COOH (1),
wobei in den vorstehenden allgemeinen Formeln (1)
**R**^{**1**} eine 2-Chlorethyl-, oder eine 2-Bromethylgruppe,
**n** eine ganze Zahl im Wert von 1, 2 oder 3,
**Ar** ein substituiertes oder unsubstituiertes Arylen oder Heteroarylen und
**X** eine direkte Bindung oder eine Ethenylgruppe bedeuten.

6. Verwendung der 3-Halogenpropionsäureester gemäß Anspruch 5 zur Herstellung von flüssigkristallinen polymerisierbaren Verbindungen und zur Herstellung von polymerisierbaren Dotierstoffen für flüssigkristalline Verbindungen zur Induzierung der cholesterischen Phase.

7. Verwendung der 3-Halogenpropionsäureester gemäß Anspruch 5 zur Herstellung von Acrylsäureestern aromatischer Hydroxycarbonsäuren der allgemeinen Formel (X)
(H₂C=CH-COO)ₙ-Ar-X-COOH (1),
in der **n, Ar** und **X** die bei Anspruch 5 aufgeführten Bedeutungen aufweisen,
wobei die 3-Halogenpropionsäureester aromatischer Hydroxycarbonsäuren der allgemeinen Formel (1) mit einer Base umgesetzt werden.

## Claims

1. Process for the preparation of esters of aromatic hydroxycarboxylic acids of the general formula (1)
(R¹-COO)ₙ-Ar-X-COOH (1),
in which, in a first step, aromatic hydroxycarboxylic acid of the general formula (2)
(HO)ₙ-Ar-X-COOH (2),
is esterified in aqueous solution in the presence of a water-miscible organic solvent and in the presence of a water-soluble or water-miscible base by 3-chloro- or 3-bromopropionyl halide to give the base salt of the esters of aromatic hydroxycarboxylic acids of the general formula (1) and,
in a second step, the base salt of the esters of aromatic hydroxycarboxylic acids of the general formula (1) is converted into the free ester of aromatic hydroxycarboxylic acids of the general formula (1) by addition of acid,
in the above general formulae (1) and (2)
R¹ denoting a 2-chloroethyl, a 2-bromoethyl or an ethenyl group,
n denoting an integer having the value of 1, 2 or 3,
Ar denoting a substituted or unsubstituted arylene or heteroarylene and
X denoting a direct bond or an ethenyl group.

2. Process according to Claim 1, in which the water-soluble base is selected from alkali metal carbonate, alkali metal hydroxide, alkaline earth metal carbonate, alkaline earth metal hydroxide and water-miscible alkylamines and amino alcohols.

3. Process according to Claim 2, in which the water-miscible solvent is selected from methanol, ethanol, isopropanol and acetone.

4. Process according to any of Claims 1 to 3, in which the acid used is a mineral acid.

5. 3-Halopropionic acid esters of aromatic hydroxycarboxylic acids of the general formula (1)
(R¹-COO)ₙ-Ar-X-COOH (1)
where, in the above general formula (1),
R¹ denotes a 2-chloroethyl or a 2-bromoethyl group,
n denotes an integer having the value of 1, 2 or 3,
Ar denotes a substituted or unsubstituted arylene or heteroarylene and
X denotes a direct bond or an ethenyl group.

6. Use of the 3-halopropionic acid esters according to Claim 5 for the preparation of liquid crystalline polymerizable compounds and for the preparation of polymerizable dopants for liquid crystalline compounds for inducing the cholesteric phase.

7. Use of the 3-halopropionic acid esters according to Claim 5 for the preparation of acrylic acid esters of aromatic hydroxycarboxylic acids of the general formula (X)
(H₂C=CH-COO)ₙ-Ar-X-COOH (X)
in which n, Ar and X have the meanings mentioned in Claim 5,
the 3-halopropionic acid esters of aromatic hydroxycarboxylic acids of the general formula (1) being reacted with a base.

## Revendications

1. Procédé de préparation d'esters d'acides hydroxycarboxyliques aromatiques de formule générale (1)
(R¹-COO)ₙ-Ar-X-COOH (1),
dans lequel, dans une première étape, un acide hydroxycarboxylique aromatique de formule générale (2)
(HO)ₙ-Ar-X-COOH (2),
est estérifié en solution aqueuse, en présence d'un solvant organique miscible à l'eau et en présence d'une base hydrosoluble ou miscible à l'eau, par l'halogénure d'acide 3-chloro- ou 3-bromopropionique pour donner lieu au sel basique des esters d'acides hydroxycarboxyliques aromatiques de formule générale (1), et
dans une deuxième étape, le sel basique des esters d'acides hydroxycarboxyliques aromatiques de formule générale (1) est transformé en ester libre d'acides hydroxycarboxyliques aromatiques de formule générale (1) par addition d'acide,
où dans les formules générales (1) et (2) ci-dessus
R¹ représente un groupe 2-chloroéthyle, 2-bromoéthyle ou éthényle,
n représente un entier valant 1, 2 ou 3,
Ar représente un arylène ou un hétéroarylène substitué ou non substitué et
X représente une liaison directe ou un groupe éthényle.

2. Procédé selon la revendication 1, dans lequel la base hydrosoluble est choisie parmi un carbonate de métal alcalin, un hydroxyde de métal alcalin, un carbonate de métal alcalinoterreux, un hydroxyde de métal alcalinoterreux et des alkylamines et amino-alcools miscibles à l'eau.

3. Procédé selon la revendication 2, dans lequel le solvant miscible à l'eau est choisi parmi le méthanol, l'éthanol, l'isopropanol et l'acétone.

4. Procédé selon les revendications 1 à 3, dans lequel un acide minéral est utilisé en tant qu'acide.

5. Esters 3-halogénopropioniques d'acides hydroxycarboxyliques aromatiques de formule générale (1)
(R¹-COO)ₙ-Ar-X-COOH (1),
où dans la formule générale (1) ci-dessus
R¹ représente un groupe 2-chloroéthyle ou 2-bromoéthyle,
n représente un entier valant 1, 2 ou 3,
Ar représente un arylène ou un hétéroarylène substitué ou non substitué et
X représente une liaison directe ou un groupe éthényle.

6. Utilisation des esters 3-halogénopropioniques selon la revendication 5, pour la préparation de composés polymérisables cristallins liquides et pour la préparation de substances dopantes polymérisables pour des composés cristallins liquides en vue d'induire la phase cholestérique.

7. Utilisation des esters 3-halogénopropioniques selon la revendication 5, pour la préparation d'esters acryliques d'acides hydroxycarboxyliques aromatiques de formule générale (X)
(H₂-C=CH-COO)ₙ-Ar-X-COOH (X),
dans laquelle n, Ar et X présentent les significations indiquées dans la revendication 5,
les esters 3-halogénopropioniques d'acides hydroxycarboxyliques aromatiques de formule générale (1) étant mis à réagir avec une base.
